Europäisches Patentamt

European Patent Office

Office européen des brevets

(11) Publication number: **0 179 958**
**B1**

(12) **EUROPEAN PATENT SPECIFICATION**

(45) Date of publication of patent specification: **17.05.89**

(21) Application number: **84307519.3**

(22) Date of filing: **31.10.84**

(51) Int. Cl.⁴: **A 61 M 1/00**

(54) **Intercostal tube.**

(43) Date of publication of application:
**07.05.86 Bulletin 86/19**

(45) Publication of the grant of the patent:
**17.05.89 Bulletin 89/20**

(84) Designated Contracting States:
**BE CH DE FR GB LI NL**

(56) References cited:
**FR-A-2 041 485**
**US-A-3 225 762**
**US-A-3 506 010**

(73) Proprietor: **SHERWOOD MEDICAL COMPANY**
**1831 Olive Street**
**St. Louis, MO 63103 (US)**

(72) Inventor: **Ring, E. Murray**
**621, South New Ballas Road**
**St. Louis Missouri 63141 (US)**

(74) Representative: **Chettle, Adrian John et al**
**c/o John Wyeth & Brother Limited Huntercombe**
**Lane South**
**Taplow Maidenhead Berkshire, SL6 0PH (GB)**

Courier Press, Leamington Spa, England.

## Description

This invention relates to intercostal tubes. Hitherto intercostal tubes have been made with squared or perpendicular end faces. Such intercostal tubes are difficult to insert between a patient's ribs because of the squared end and the large area that must be inserted at once. Accordingly the end to be inserted is usually squeezed or compressed transversely to make the tube narrower and therefore require less of an incision in order to pass between the ribs than would otherwise be the case. However, squeezing the tube increases the maximum dimension of the tube end and thereby requires a larger opening in the area between the ribs which can cause the patient pain.

FR—A—2 041 485 proposes a solution in which the intercostal tube is inserted from the outside using a hardened point. To avoid damage to the patient the point is cut off immediately after insertion. This necessarily requires a second opening, rather larger than the first, which indicates that the tube is intended for use after major surgery. The main surgical opening can be used to view the point as it passes through the chest wall, the tip being cut off immediately before the main surgical opening is closed.

US—A—3 190 290 discloses a tube which is pulled through the chest wall from the inside using the main surgical opening for access. The pointed end cannot damage the patient internally and the tube can be soft because it is inserted under tension.

The present invention does not require a second surgical opening and yet avoids injury to the patient because the tip is blunt. The prior art teaches very sharp points, to avoid patient pain, and very stiff points to avoid tip bending.

According to the invention there is provided an intercostal tube of somewhat flexible plastic material, the tube being of a size to enable drainage from a body cavity into which it is inserted, and being flexible about its longitudinal axis but relatively resistant to transverse crushing force; the tube having a somewhat flexible sloping tip end portion (12) for insertion into the body by way of a passage therein made such as one between the ribs; the end of the tube being blunted to reduce its tendency to bend or to snag body tissue during insertion, the sloping tip (12), when viewed transversely of the tube axis, extending in a single slope from a point on the surface of the tube toward the end of the tube and toward the opposite side of the tube but terminating short of the opposite side of the tube, leaving a continuous end (13) that is blunted and that extends around the tube, and the blunted part of which when viewed from the end the tube, is arcuate and is relatively rigid against collapse upon being forced through a passage such as the one between the ribs.

Such an intercostal tube has the advantage that the bulging of the tube end does not significantly increase the maximum transverse dimension of the tube. The tube may thus be inserted between the ribs of a patient with less pain than prior art intercostal tubes.

Preferably the sloping end is substantially planar; the slope may be at twenty five degrees to the longitudinal axis of the tube for example.

The sloping end terminates in a rounded, relatively blunt lip. This construction gives increased rigidity and resistance to bending under axial pressure than if the sloping edge extended entirely across the tube and the lip were pointed.

A sloping end may be provided at each end of the intercostal tube.

Other features of the invention wll be apparent from the following description of a preferred embodiment shown, by way of example only, with reference to the accompanying drawings in which:

Fig. 1 is a broken isometric view of an intercostal tube according to the invention;

Fig. 2 is a plan view of the tube of Fig. 1;

Fig. 3 is an end view of a prior art intercostal tube with the tube end squeezed to allow for entrance between a patient's ribs;

Fig. 4 is an end view of the tube of Fig. 1 and corresponding to Fig. 3.

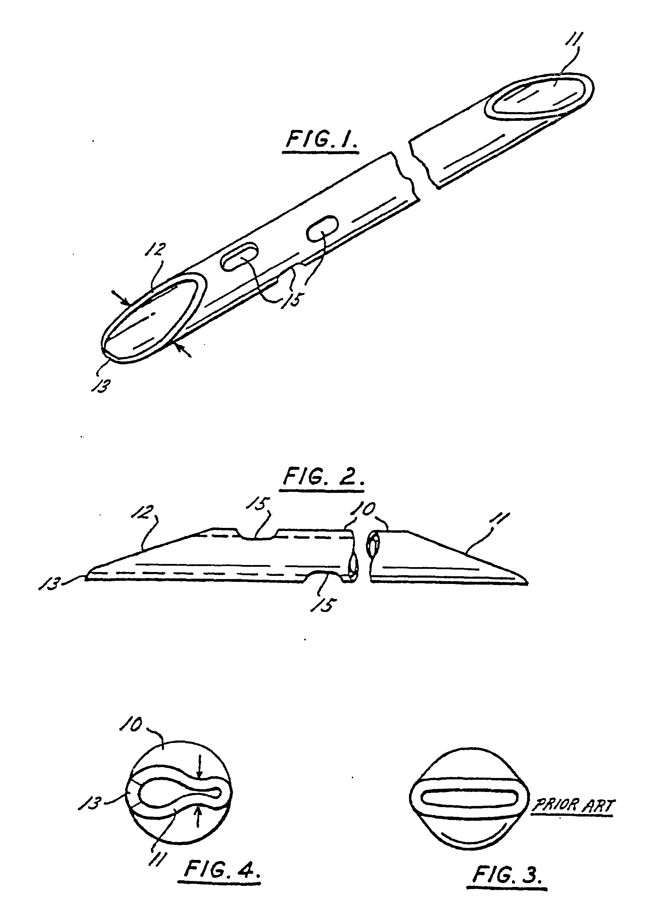

With reference to Figs 1 and 2 here is shown an intercostal tube 10 having a discharge end 11. The other end has an acute sloping edge 12 that is substantially planar and disposed at approximately twenty five degrees to the longitudinal axis of the tube. In the preferred embodiment the sloping edge 12 does not extend entirely across the tube but terminates in an end edge or lip 13 which is arcuate and thereby stronger against bending under axial pressure than the end of the tube would be if the sloping edge 12 went entirely across the tube and the lip were nearly pointed.

The tube has drainage holes 15 spaced from the sloping edge 12 to admit fluids from the body after insertion; these fluids can drain out at the discharge end 11.

The tube may be made of one of the polyhalogenated polyethylenes or an equivalent plastic material suitable for medical use. The material may be for example Tygon (trade mark) or Teflon (trade mark); these materials are flexible but rather resistant to distortion transversely of the tube. The tubes are normally about 450—500mm long, to 40 French diameter, with walls approximately 2mm thick for a tube approximately 14mm outside diameter, or 40 French.

In use the tube of the present invention can be inserted between a patient's ribs into the thoracic area. Since the lip 13 is somewhat blunt and arcuate in shape, the end of the tube will not collapse or bend over. The steep slope 12 permits the tube to be inserted between the ribs with the minimum size of opening. Usually a clamp is applied to the end of the tube for insertion as indicated by arrows in Figs. 1 and 4. Because of the sloping edges and cut-away sides the squeezing applied along the sloping end does not bulge parts of the tube significantly beyond the diameter of the tube. As shown in Fig. 3 the lateral extension of a squared tube when squeezed is beyond the nominal diameter of the tube. Such lateral extension or bulging causes pain to the patient.

The tube remains flexible so that its axis can be bent to conform to conditions of use.

Various changes and modifications may be made to the invention as would be apparent to those skilled in the art. It is intended that the invention be limited only by the scope of the Claims appended hereto.

## Claims

1. An intercostal tube of somewhat flexible plastic material, the tube being of a size to enable drainage from a body cavity into which it is inserted, and being flexible about its longitudinal axis but relatively resistant to transverse crushing force; the tube having a somewhat flexible sloping tip end portion (12) for insertion into the body by way of a passage therein made such as one between the ribs; the end of the tube being blunted to reduce its tendency to bend or to snag body tissue during insertion, the sloping tip (12), when viewed transversely of the tube axis, extending in a single slope from a point on the surface of the tube toward the end of the tube and toward the opposite side of the tube but terminating short of the opposite side of the tube, leaving a continuous end (13) that is blunted and that extends around the tube, and the blunted part of which when viewed from the end of the tube, is arcuate and is relatively rigid against collapse upon being forced through a passage such as the one between the ribs.

2. The tube of claim 1, the slope being about twenty-five degrees to the longitudinal axis, and the tip of the tube being rigid enough to be inserted between the ribs without bending.

## Patentansprüche

1. Intercostale Röhre aus etwas flexiblem Kunststoff, wobei die Röhre so bemessen ist, daß die Drainage einer Körperhöhle, in welche sie eingesetzt wird, möglich ist, und die in der Längsachse flexibel aber relativ widerstandsfähig gegen Querverformungskräfte ist; wobei die Röhre ein etwas flexibles abgeschrägt spitzes Endstück (12) zum Einführen in den Körper durch eine darin vorgesehene Öffnung, z. B. zwischen den Rippen, aufweist; wobei das Ende der Röhre stumpfkantig ist, um seine Neigung beim Einführen das Körpergewebe zu verbiegen und sich darin festzusetzen, zu verringern; wobei die Abschrägung der Spitze (12), quer zur Achse der Röhre betrachtet, von einem Punkt an der Oberfläche der Röhre zum Röhrenende und der gegenüberliegenden Seite der Röhre hin in einer einzigen Abschrägung verläuft, jedoch kurz vor der gegenüberliegenden Seite der Röhre endet und dabei ein auslaufendes stumpfkantiges Ende (13) bildet, das das Rohr umfaßt, wobei der stumpfkantige Teil, vom Rohrende her gesehen, bogenförmig und relativ kollabierungsfest ist, wenn man ihn durch eine Öffnung, z. B. zwischen den Rippen, schiebt.

2. Röhre nach Anspruch 1, wobei die Schräge ungefähr 25°, bezogen auf die Längsachse, beträgt, und die Spitze der Röhre so steif ist, daß sie ohne Verbiegung zwischen den Rippen eingeführt werden kann.

## Revendications

1. Tube intercostal en matière plastique légèrement flexible, le tube étant d'un calibre destiné à permettre le drainage d'une cavité du corps dans laquelle il est inséré, et étant flexible autour de son axe longitudinal, mais relativement résistant à une force d'écrasement transversale; le tube présentant une partie d'extrémité taillée en sifflet légèrement flexible (12) à insérer dans le corps par un passage qui y est ménagé, par exemple un passage entre les côtes; l'extrémité du tube étant rendue mousse pour diminuer sa tendance à fléchir ou à accrocher des tissus corporels pendant l'insertion, l'extrémité en sifflet (12), lorsqu'elle est vue transversalement à l'axe du tube, s'étendant suivant une pente simple depuis un point situé sur la surface du tube vers l'extrémité de ce tube et vers le côté opposé de celuici, mais se terminant à courte distance de ce côté opposé, de manière à laisser une extrémité continue (13) qui est mousse et qui s'étend tout autour du tube, et la partie mousse de cette extrémité, dans une vue en bout du tube, est courbe et est relativement rigide de manière à ne pas s'aplatir lorsqu'elle est enfoncée à travers un passage tel que le passage prévu entre les côtes.

2. Tube suivant la revendication 1, la pente étant d'environ 25° par rapport à l'axe longitudinal et l'extrémité du tube étant suffisamment rigide pour pouvoir être insérée entre les côtes sans fléchir.

*FIG. 1.*

*FIG. 2.*

*FIG. 4.*

*FIG. 3.*

PRIOR ART